Europäisches Patentamt

European Patent Office

Office européen des brevets

(19)

(11) Publication number : **0 636 373 A1**

(12) **EUROPEAN PATENT APPLICATION**

(21) Application number : **94500127.9**

(22) Date of filing : **19.07.94**

(51) Int. Cl.[6] : **A61K 35/78**

(30) Priority : **30.07.93 ES 9301798**

(43) Date of publication of application :
**01.02.95 Bulletin 95/05**

(84) Designated Contracting States :
**DE ES FR GB IT**

(71) Applicant : **INKEYSA, S.A.**
**15-19, calle Juan XXIII**
**E-08950 Esplugas de Llobregat (ES)**

(72) Inventor : **Perez Carlos, Francisco**
**15-19, calle Juan XXIII**
**E-08950 Esplugas de Llobregat (ES)**

(74) Representative : **SUGRANES - VERDONCES -**
**FERREGÜELA**
**Calle Provenza, 304**
**E-08008 Barcelona (ES)**

(54) **Compositions comprising Plantago Mayor, Rosa Canina and Citrus Aurantium for treating psoriasis.**

(57)    New compositions for the effective treatment of psoriasis, containing no mercury salts and based on the combination of distilled aqueous extracts of plantain (Plantago major L.), wild rose (Rosa canina L.) and bitter orange (Citrus aurantium L.).

Also described is a procedure for obtaining said compositions by mixing the vegetable extracts, eliminating part of the total volume by distillation and compensating the volume distilled with 96° alcohol.

The new compositions described are highly effective in treating the symptoms of the disease, they reduce the risk of relapses and furthermore exhibit excellent tolerance.

EP 0 636 373 A1

The present invention relates to therapeutic compositions for topical use for the treatment of psoriasis, based on the combination of distilled aqueous extracts of vegetables, and a procedure for obtaining them.

## BACKGROUND OF THE INVENTION

Psoriasis is a common, chronic and recurring disease, characterized by the formation of dry and well-defined patches and silvery peeling scales of different sizes on certain areas of the epidermis. It characteristically affects the scalp, the extensor surfaces of the extremities, in particular the elbows and knees, the back and the buttocks, as well as the nails, eyebrows, axillae, naval and anogenital region. Occasionally the disease is generalized.

Its causes are unknown and therefore current forms of treatment are of the symptomatic type. An effective treatment is therefore considered to be one which achieves a high percentage of remission of the lesions over a relatively long period without the reappearance of said lesions.

There are various medicines for treating psoriasis but which in general involve problems and complications. For example, methotrexate and etretinate are too toxic to for continued preventative treatment. Salicylic acid is only effective in mild cases. Coal tar, which is normally used together with zinc oxide, is often not accepted by the patient due to its unpleasant smell and the fact that it is difficult to apply. Ditranol, despite its effectiveness, is irritating to the epidermis and produces stains on the clothes, nails and skin. The corticoids for topical use suffer from the drawback of tachyphylaxis and furthermore when treatment is stopped the symptoms suddenly reappear. Finally, ultra-violet radiation treatment has problems associated with its long term effects, such as premature aging of the skin and the increased risk of skin cancer.

The use of extracts of natural origin is also known in the topical treatment of the disease. The PCT patent application number 89/05651 proposes the use of oily extracts of a collection of plants such as garlic, the common nettle and veronica, etc. The European patent application number 367103 describes the use of ointments made with jojoba and beeswax or spermaceti. Finally, the Spanish patent number 2.006.474 describes the use of extracts of plants belonging to the family of the zygophyllaceae together with an aqueous solution of a non-alkali metal halide.

There are thus many methods of treatment but none of them are sufficiently safe and effective and as a result there is a need for an effective treatment for psoriasis which does not involve residual toxic effects nor the rapid occurrence of relapses and which at the same time is readily accepted by the patient by not being unpleasant to apply.

The authors of the present invention have filed the Spanish patent application number 9202309 and the French patent application number 9205159, neither of which have yet been published, which describe compositions for the treatment of psoriasis based on the combined use of distilled aqueous extracts of the leaves and spikes of plantain (Plantago major L.), the petals of wild rose (Rosa canina L.) and the flowers of bitter orange (Citrus aurantium L.) together with an organic or inorganic mercury salt. Said compositions, despite containing mercury salts, are only moderately toxic.

It has now been discovered, surprisingly, that the right combination of the vegetable extracts mentioned above, without mercury salts, exhibits excellent properties in the topical treatment of psoriasis and is even less toxic than the compositions described in the above mentioned patent applications pending publication.

This constitutes unquestionable technical progress since the elimination of mercury salts in topical therapeutic treatments is clearly desirable in order to avoid problems of toxicity, in particular in the treatment of chronic cases or cases in which high doses and frequent applications are required.

## DESCRIPTION OF THE INVENTION

The object of the present invention is to provide aqueous compositions of vegetable extracts which can be used for the effective topical treatment of psoriasis without the need to use mercury salts, and therefore with a very low degree of toxicity, which reduce as much as possible the occurrence of relapses.

The compositions for the topical treatment of psoriasis which form the object of the present invention consist essentially of the combination of distilled aqueous extracts of the leaves and spikes of plantain (Plantago major L.), the petals of wild rose (Rosa canina L.) and the flowers of bitter orange (Citrus aurantium L.).

Said extracts, commonly known as plantain water, rose water and orange flower water, are obtained separately by soaking the parts of the plants mentioned above in water and then distilling the extract, collecting the distillate as a useful substance for the purposes of the present invention.

The plantain water, rose water and orange flower water can be prepared in situ or can be purchased.

In order to obtain the excellent therapeutic properties against psoriasis described above, the compositions that form the object of the present invention must contain the three types of vegetable extract: plantain water, rose water and orange flower water.

Once the mixture of components has been made it is preferable to eliminate between 5 and 20% of the total volume of liquid by distillation and compensate the volume distilled by adding an equivalent volume of 96° ethanol.

Therefore, the procedure may consist of mixing the three distilled aqueous extracts of plantain, rose and orange flower, eliminating between 5 and 20% of the volume by distillation at atmospheric pressure, compensating the volume distilled with 96° ethanol and filtering until the desired composition is obtained.

Good therapeutic results are achieved when the essential components are present in the following proportions:

| | |
|---|---|
| Plantain water | 45-60 % (V/V) |
| Rose water | 20-35 % (V/V) |
| Orange flower water | 10-20 % (V/V) |

relative to the total volume of the mixture before the reduction in volume by distillation, where (V/V) indicates proportion by volume.

The best therapeutic results in terms of effectiveness are achieved when the proportion of plantain water, expressed by volume as described above, is greater than 50%, that of rose water less than 30% and that of orange flower water greater than 12%. Therefore, said proportions are particularly preferable.

Surprisingly, it has been discovered that although they do not contain mercury salts, the compositions of the present invention are highly effective in the topical treatment of psoriasis, without exhibiting the toxicity associated with said salts.

It has been shown by means of clinical experiments that the application of the compositions of the present invention onto the psoriatic scales of different types of patient causes said scales to fall away by returning to normal the cell renewal cycle of the skin, which recovers its natural elasticity.

It has been further shown that relapses reduce considerably and have much less acute symptoms, and that prolonged treatment leads to a total cure.

Furthermore, the compositions that form the object of the present invention are innocuous, have a pleasant smell of roses and are non-irritant, and as such are readily accepted by the patient.

In order that the present invention be better understood, the following non-limiting examples are described.

## EXAMPLES

### Example 1

The plantain, rose and orange flower waters are obtained by soaking the plant powder at room temperature for 10-24 hours in 600-800 ml of distilled water for every 100 g of plant powder. The resulting liquid is subjected to a simple distillation to obtain a distillate which has 60% of the original volume.

The following amounts of the distilled aqueous extracts are mixed in a suitable receptacle:

| | |
|---|---|
| Plantain water | 1700 ml |
| Rose water | 810 ml |
| Orange flower water | 450 ml |

The volume is then made up to three litres with distilled water.

The solution obtained is distilled at 100°C for 15 minutes, giving a resulting volume of about 2.7 litres which are left to cool. 300 ml of 96° ethanol are added and the solution is filtered.

The solution is left to stand for 24 hours after which it is filtered again and is ready for use.

### Example 2

The following amounts of the distilled aqueous extracts, obtained as described in example 1, are mixed in a suitable receptacle:

EP 0 636 373 A1

| Plantain water | 1620 ml |
|---|---|
| Rose water | 810 ml |
| Orange flower water | 450 ml |

The volume is then made up to three litres with distilled water.

The solution obtained is distilled at 100°C for 15 minutes, giving a resulting volume of about 2.7 litres which are left to cool. 300 ml of 96° ethanol are added and the solution is filtered.

The solution is left to stand for 24 hours after which it is filtered again and is ready for use.

Example 3

The following amounts of the distilled aqueous extracts, obtained as described in example 1, are mixed in a suitable receptacle:

| Plantain water | 1500 ml |
|---|---|
| Rose water | 1050 ml |
| Orange flower water | 360 ml |

The volume is then made up to three litres with distilled water.

The solution obtained is distilled at 100°C for 15 minutes, giving a resulting volume of about 2.7 litres which are left to cool. 300 ml of 96° ethanol are added and the solution is filtered.

The solution is left to stand for 24 hours after which it is filtered again and is ready for use.

Example 4

The following amounts of the distilled aqueous extracts, obtained as described in example 1, are mixed in a suitable receptacle:

| Plantain water | 1350 ml |
|---|---|
| Rose water | 960 ml |
| Orange flower water | 450 ml |

The volume is then made up to three litres with distilled water.

The solution obtained is distilled at 100°C for 15 minutes, giving a resulting volume of about 2.7 litres which are left to cool. 300 ml of 96° ethanol are added and the solution is filtered.

The solution is left to stand for 24 hours after which it is filtered again and is ready for use.

Example 5

Clinical tests are carried out on the compositions obtained in the previous examples.

50 patients are treated by means of applications every 12 hours, preferably in the mornings and at night, until the whole whitening.

The results obtained are shown in table 1.

4

Table 1

| Composition | Effectiveness | Tolerance |
|---|---|---|
| Example 1 | ++++ | ++++ |
| Example 2 | +++ | ++++ |
| Example 3 | +++ | ++++ |
| Example 4 | ++ | ++++ |

Each composition is evaluated by assigning crosses according to the results observed.

All of the compositions are effective and have an excellent level of tolerance but, as can be seen in the table, the behaviour of the composition obtained in example 1 is particularly noteworthy due its greater effectiveness.

Table 2 shows in more detail the results obtained with a composition similar to that of example 1.

Table 2

| Type of Psoriasis | No. of Patients | Duration of Psoriasis (years) | % of Area Affected | Whitening Time (months) |
|---|---|---|---|---|
| General | 39 | 1-17 | 5-85 | 1-4 |
| Palm/sole | 3 | 4-25 | 3-5 | 2-4 |
| Guttate | 5 | 3-19 | 15-99 | 2-5 |
| Pustular | 3 | 2-7 | 10-99 | 2-5 |
| Total range | 50 | 1-25 | 3-99 | 1-5 |

The follow-up carried out with these patients shows that after the initial whitening, 2 to 4 relapses normally appear within a maximum period of 2 years, said relapses being very reduced in area and being milder and more localized each time until the symptoms disappear completely.

After the first two years, and even after an average of three years after treatment stops, the disease does not reappear.

**Claims**

1. Therapeutic compositions containing no mercury salts for use in the treatment of psoriasis, characterized in that they consist essentially of the combination of distilled aqueous extracts of the leaves and spikes of plantain (Plantago major L.), the petals of wild rose (Rosa canina L.) and the flowers of bitter orange (Citrus aurantium L.).

2. Therapeutic compositions according to claim 1, characterized in that they are prepared by mixing the following components in the proportions shown:

| Plantain water | 45-60 % (V/V) |
|---|---|
| Rose water | 20-35 % (V/V) |
| Orange flower water | 10-20 % (V/V) |

relative to the total volume of the mixture, where (V/V) indicates proportion by volume, followed by the elimination by distillation of between 5 and 20% of the initial volume of the mixture, compensating the volume distilled with an equal volume of 96° ethanol.

3. Therapeutic compositions according to claim 2, characterized in that the proportion by volume of plantain water in the initial mixture is greater than 50%

EP 0 636 373 A1

**European Patent Office**

# EUROPEAN SEARCH REPORT

Application Number

EP 94 50 0127

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 297 640 (IRIS SAS) * the whole document * | 1-3 | A61K35/78 |
| A | DE-A-36 15 793 (BEESE JOSEF) * the whole document * | 1-3 | |

TECHNICAL FIELDS SEARCHED (Int.Cl.6)

A61K

The present search report has been drawn up for all claims

| Place of search | Date of completion of the search | Examiner |
|---|---|---|
| THE HAGUE | 11 October 1994 | Fernandez y Branas,F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6